# EUROPEAN PATENT SPECIFICATION

(11) **EP 3 116 424 B1**
(45) Date of publication and mention of the grant of the patent: **12.06.2019**
(21) Application number: 15734485.4
(22) Date of filing: 11.03.2015
(51) Int. Cl.: A61B 17/80, A61B 17/82, A61B 17/88

(54) **STERNAL CLOSURE CERCLAGE, PLATE IMPLANT AND INSTRUMENTATION**
UMSCHLINGUNGSDRAHT ZUM VERSCHLIESSEN DES BRUSTBEINS, PLATTENIMPLANTAT UND INSTRUMENTE
CERCLAGE DE FERMETURE STERNALE, IMPLANT DE PLAQUE, ET INSTRUMENTATION

(30) Priority: 14.03.2014 US 201461953216 P; 22.08.2014 US 201414466485
(43) Date of publication of application: 18.01.2017
(73) Proprietor: ZIMMER BIOMET CMF AND THORACIC, LLC, Jacksonville, FL 33218 (US)
(72) Inventor: GARCIA, Saddy R., St. Augustine, Florida 32086 (US); LUBY, Ryan N., Ponte Vedra Beach, Florida 32082 (US); WILCOX, Bryan, St. Augustine, Florida 32092 (US); SIDWELL, Scott, Jacksonville, Florida 32218 (US); BRUNEAU, Aurelien, Jacksonville, Florida 32224 (US); SINK, Sam, Neptune Beach, Florida 32266 (US); HALE, Rachel, Jacksonville, Florida 32223 (US); WITTEN, Benjamin, Jacksonville, Florida 32216 (US)
(74) Representative: Mays, Julie
(86) International application number: PCT/US2015/019976
(87) International publication number: WO 2015/142588

(56) References cited:
- WO-A1-95/22294
- WO-A1-03/037196
- WO-A1-2009/100339
- DE-A1- 4 021 246

## Description

### FIELD

The present disclosure relates to sternal closure devices and instrumentation.

### BACKGROUND

This section provides background information related to the present disclosure and is not necessarily prior art.

Surgeries are often performed on humans and animals to treat disease or injury. Such surgeries can result in the surgeon having to repair a separated, cut or fractured bone. In the case of an injury, the surgeon may need to immobilize and fix two or more bone portions together to allow the bone to heal over time. Treating certain diseases, such as heart disease, for example, often requires the surgeon to cut a patient's sternum to gain access to and perform a procedure on the patient's heart. Once the heart procedure is complete, the cut sternum may be repaired by immobilizing and joining the separate portions of the sternum together to allow the sternum to heal over time. It is often desirable to apply a compressive force urging the bone portions together to facilitate healing of the bone.

### SUMMARY

This section provides a general summary of the disclosure, and is not a comprehensive disclosure of its full scope or all of its features.

In one form, the present invention provides a closure system that secures a first bone portion to a second bone portion. The closure system includes a band, a locking terminal, and a tensioning device. The band is adapted to be looped around the first and second bone portions. The locking terminal fixedly engages a first portion of the band and selectively fixedly engages a second portion of the band. The tensioning device includes a body and a threaded rod. The threaded rod is received in the body and engages the band such that movement of the threaded rod relative to the body moves the second portion of the band relative to the first portion of the band.

In some embodiments, the closure system may also include a bracket engaging the band. The bracket can be fastened to one or both of the bone portions. The band and the bracket may be implantable with a human body. The first and second bone portions may be first and second portions of a sternum.

In some embodiments, the tensioning device includes a sleeve that slidably receives the body.

In some embodiments, the tensioning device includes a resiliently flexible collar received in the sleeve and receiving a portion of the body.

In some embodiments, the collar includes a first axial end engaging an annular groove in the body and a second axial end that removably engages the locking terminal.

In some embodiments, the sleeve is axially movable relative to the body and the collar between a first position restricting radial flexing of the collar and a second position allowing the collar to flex radially outward relative to the body.

In some embodiments, the tensioning device includes an adjustment member partially received in the body and threadably engaging the threaded rod, the adjustment member is rotatable relative to the body and is axially fixed relative to the body such that rotation of the adjustment member relative to the body causes corresponding axial movement of the threaded rod relative to the body.

In some embodiments, the tensioning device includes an anti-rotation pin extending through the body and engaging the threaded rod to restrict relative rotation between the threaded rod and the body while allowing relative axial movement between the threaded rod and the body.

In some embodiments, the tensioning device includes a toggle lock mounted to the threaded rod and movable relative to the threaded rod between an unlocked position allowing the band to move through an aperture in the threaded rod and a locked position in which the toggle lock engaging the band and restricts movement of the band relative to the threaded rod.

In some embodiments, the locking terminal includes a casing and a cam member. The casing may engage the first portion of the band. The cam member may be rotatable relative to the casing and the first portion of the band between an unlocked position allowing the second portion of the band to move through the casing and a locked position in which the cam member engages the second portion of the band to restrict movement of the second portion relative to the casing and the first portion.

In some embodiments, the casing includes a first casing member and a second casing member. The first portion of the band and the cam member may be disposed between the first and second casing members.

In some embodiments, the first and second casing members and the first portion of the band all include apertures that are aligned with each other to allow the second portion of the band to pass therethrough.

In some embodiments, the casing is a one-piece, unitary body.

In some embodiments, the cam member clamps the second portion of the band against the casing in the locked position.

In some embodiments, the first portion of the band includes a deflectable tab that allows movement of the cam member from the unlocked position to the locked position and restricts movement of the cam member from the locked position to the unlocked position.

In some embodiments, the cam member includes a deflectable tab that releasably engages the casing in the locked position to restrict the cam member from moving out of the locked position.

In some embodiments, the deflectable tab releasably engages a first aperture (e.g., a hole, recess, notch or slot) in the casing member in the locked position to restrict movement of the cam member out of the locked position. The deflectable tab may releasably engage a second aperture (e.g., a hole, recess, notch or slot) in the casing in the unlocked position to restrict movement of the cam member out of the unlocked position.

In some embodiments, the body includes a member fixed to an axial end of the body. The member may include a rim defining a central recess receiving the locking terminal. The rim may include a keyway receiving a portion of the cam member such that relative rotation of the body relative to the locking terminal causes rotation of the cam member from the unlocked position to the locked position.

In some embodiments, the member includes a shearing aperture through which the second portion of the band extends. Walls of the shearing aperture may be configured to cut the second portion of the band in response to rotation of the body relative to the locking terminal.

In some embodiments, the shearing aperture is configured so that the walls of the shearing aperture will cut the second portion of the band only after at least ninety degrees of rotation of the body relative to the locking terminal.

In some embodiments, the shearing aperture is a bowtie-shaped aperture.

Also disclosed is a tensioning device for tightening a band around a first bone portion and a second bone portion. The tensioning device may include an elongated body, a threaded rod, a sleeve and a resiliently flexible locking arm. The elongated body may include a central aperture extending axially through the body. The threaded rod may be received in the central aperture of the body and may be adapted to engage the band such that movement of the threaded rod relative to the body moves a second portion of the band relative to a first portion of the band. The sleeve may slidably receive the body. The locking arm may extend from the body and may be configured to releasably secure the tensioning device relative to the band.

In some embodiments, the locking arm is a part of a collar received in the sleeve and receiving a portion of the body.

In some embodiments, the collar includes an axial end engaging an annular groove in the body.

In some embodiments, the sleeve is axially movable relative to the body and the locking arm between a first position restricting radial flexing of the locking arm and a second position allowing the locking arm to flex radially outward relative to the body.

In some embodiments, the tensioning device includes an adjustment member partially received in the body and threadably engaging the threaded rod. The adjustment member may be rotatable relative to the body and may be axially fixed relative to the body such that rotation of the adjustment member relative to the body causes corresponding axial movement of the threaded rod relative to the body.

In some embodiments, the tensioning device includes an anti-rotation pin extending through the body and engaging the threaded rod to restrict relative rotation between the threaded rod and the body while allowing relative axial movement between the threaded rod and the body.

In some embodiments, the tensioning device includes a toggle lock mounted to the threaded rod and movable relative to the threaded rod between an unlocked position allowing the band to move through an aperture in the threaded rod and a locked position in which the toggle lock engaging the band and restricts movement of the band relative to the threaded rod.

In some embodiments, the body includes a shearing member fixed to an axial end of the body, the shearing member including a rim defining a central recess and a keyway extending through the rim.

In some embodiments, the shearing member includes a shearing aperture extending from the central recess of the shearing member to the central aperture of the body.

In some embodiments, the shearing aperture is a bowtie-shaped aperture.

The tensioning device may be incorporated into a closure system that includes a locking terminal having a first casing member, a second casing member and a cam member. A first portion of the band and the cam member are disposed between the first and second casing members.

In some embodiments, the closure system may also include a bracket engaging the band. The bracket can be fastened to one or both of the bone portions. The band and the bracket may be implantable with a human body. The first and second bone portions may be first and second portions of a sternum.

In some embodiments, the first and second casing members and the first portion of the band all include apertures that are aligned with each other to allow a second portion of the band to pass therethrough.

In some embodiments, the cam member is rotatable relative to the first and second casing members and the first portion of the band between an unlocked position allowing the second portion of the band to move through the apertures of the first and second casing members and the aperture of the first portion and a locked position in which the cam member engages the second portion of the band to restrict movement of the second portion through the apertures of the first and second casing members and the aperture of the first portion.

In some embodiments, the cam member clamps the second portion of the band against the second casing member in the locked position.

In some embodiments, the first portion of the band includes a deflectable tab that allows movement of the cam member from the unlocked position to the locked position and restricts movement of the cam member from the locked position to the unlocked position.

In some embodiments, the central recess of the shearing member receives the locking terminal. The keyway of the shearing member may receive a portion of the cam member such that relative rotation of the body relative to the locking terminal causes rotation of the cam member from the unlocked position to the locked position.

In some embodiments, the second portion of the band extends through the shearing aperture. Walls of the shearing aperture may be configured to cut the second portion of the band in response to rotation of the body relative to the locking terminal.

In some embodiments, the shearing aperture is configured so that the walls of the shearing aperture will cut the second portion of the band only after at least ninety degrees of rotation of the body relative to the locking terminal.

Also disclosed is a closure system that may secure a first bone portion to a second bone portion. The closure system may include a bracket, a band and a tensioning device. The bracket may include a first portion adapted to engage the first bone portion and a second portion adapted to engage the second bone portion. The band may engage the bracket and may be adapted to be looped around the first and second bone portions. The tensioning device may include a body and a threaded member. The threaded member may be received in the body and may engage the band such that rotation of the threaded member relative to the body moves a first end of the band relative to a second end of the band.

In some embodiments, the band and the bracket are implantable with a human body.

In some embodiments, at least a portion of the tensioning device is implantable within a human body.

In some embodiments, the first and second bone portions are first and second portions of a sternum.

In some embodiments, the threaded member threadably engages the band.

In some embodiments, the body of the tensioning device fixedly engages the first end of the band.

In some embodiments, the bracket fixedly engages the first end of the band.

In some embodiments, the band includes a plurality of apertures configured to receive a thread of the threaded member.

In some embodiments, the closure system includes a cam member that is movable relative to the bracket between a first position allowing movement of the band relative to the bracket and a second position restricting movement of the band relative to the bracket.

In some embodiments, the tensioning device is removable from the bracket and the band without releasing tension of the band.

In some embodiments, the threaded member includes a threaded shaft including a slot configured to receive the band so that the band wraps around the threaded shaft during rotation of the of the threaded member relative to the body.

In some embodiments, the body includes a plurality of guide apertures that are axially aligned with apertures in the bracket.

In some embodiments, a sleeve is molded around the band and through at least one aperture in the band.

Also disclosed is a closure system for securing a first bone portion to a second bone portion. The closure system may include a bracket, a band and a tensioning device. The bracket adapted to engage at least one of the first and second bone portions. The band may engage the bracket and may be adapted to be looped around the first and second bone portions. The tensioning device may include a base and a tensioning member. The tensioning member may rotatably engage the base and may engage the band such that rotation of the tensioning member relative to the base tensions the band relative to the first and second bone portions.

In some embodiments, the band and the bracket are implantable within a human body.

In some embodiments, at least a portion of the tensioning device is implantable within a human body.

In some embodiments, the first and second bone portions are first and second portions of a sternum.

In some embodiments, the tensioning member includes a threaded member.

In some embodiments, the tensioning member threadably engages the band.

In some embodiments, the closure system includes a cam member that is movable relative to the bracket between a first position allowing movement of the band relative to the bracket and a second position restricting movement of the band relative to the bracket.

In some embodiments, the tensioning member twists the band relative to the bracket.

In some embodiments, a first end of the band is fixed relative to the bracket and a second end of the band moves relative to the first end of the band as the tensioning member rotates relative to the body.

In some embodiments, the first and second ends of the band move relative to the bracket as the tensioning member rotates relative to the body.

In some embodiments, the band includes a plurality of apertures configured to receive a thread of the tensioning member.

In some embodiments, the tensioning device is removable from the bracket and the band without releasing tension of the band.

In some embodiments, the tensioning member includes a threaded shaft including a slot configured to receive the band so that the band wraps around the threaded shaft during rotation of the of the tensioning member relative to the base.

In some embodiments, the base includes a plurality of guide apertures that are axially aligned with apertures in the bracket.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.

The claimed invention is shown in Figures 11-28.
Figure 1 is a partial perspective view of an anterior side of a sternum and ribs of a human body having a closure device attached thereto according to the principles of the present disclosure;
Figure 2 is a perspective view of the closure device;
Figure 3 is an exploded perspective view of the closure device;
Figure 4 is a perspective view depicting a tensioning device of the closure device adjustably engaging a band of the closure device;
Figure 5 is a cross-sectional view of the closure device taken along line 5-5 of Figure 4;
Figure 6 is a perspective view of the closure device after an end of the band has been trimmed according to the principles of the present disclosure;
Figure 7 is a perspective view of the closure device after the end of the band has been bent according to the principles of the present disclosure;
Figure 8 is a perspective view of the closure device and a fixture aligning a plurality of fasteners relative to the closure device;
Figure 9 is a perspective view of the closure device and fixture of Figure 8 depicting a screwdriver driving one of the fasteners;
Figure 10 is a perspective view of the closure device with all of the fasteners driven through a bracket of the closure device;
Figure 11 is a perspective view of a configuration of a closure device in a first position according to the principles of the present invention;
Figure 11a is a sectioned perspective view of a band having a sleeve according to the principles of the present invention;
Figure 12 is an exploded perspective view of the band, a bracket and a locking device of the closure device of Figure 11;
Figure 13 is an exploded perspective view of a tensioning device of the closure device of Figure 11;
Figure 14 is a cross-sectional view of the closure device of Figure 11;
Figure 15 is another cross-sectional view of the closure device of Figure 11;
Figure 16 is a perspective view of an end of the tensioning device of Figure 13;
Figure 17 is a partial plan view of the band, bracket and locking device of Figure 12;
Figure 18 is a partial perspective view of the closure device of Figure 11;
Figure 19 is a perspective view of the tensioning device in a second position;
Figure 20 is a perspective view of the tensioning device in a third position;
Figure 21 is a partial perspective view of the tensioning device in a fourth position;
Figure 22 is a partial cross-sectional view of the tensioning device in the fourth position;
Figure 23 is a perspective view of the locking device in a locked position;
Figure 24 is a cross-sectional view of the locking device in the locked position;
Figure 25 is a perspective view of another locking device in an unlocked position;
Figure 26 is another perspective view of the locking device of Figure 25 in the unlocked position;
Figure 27 is a perspective view of the locking device of Figure 25 in a locked position; and
Figure 28 is another perspective view of the locking device of Figure 25 in the locked position.

Corresponding reference numerals indicate corresponding parts throughout the several views of the drawings.

### DETAILED DESCRIPTION

Example embodiments will now be described more fully with reference to the accompanying drawings.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

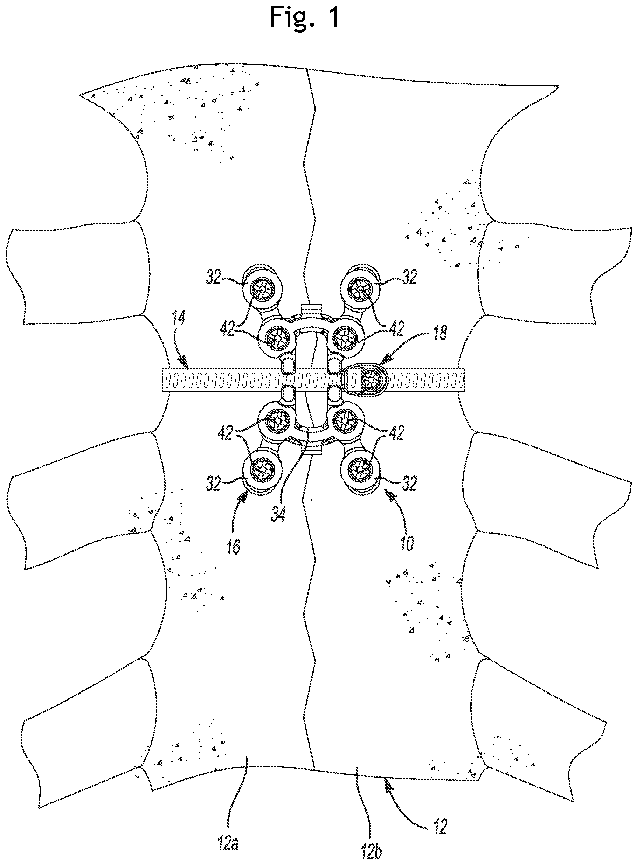

With reference to Figure 1, a closure system according to the principles of the present disclosure is generally identified with reference numeral 10. The system 10 is depicted operatively associated with a sternum 12 of a human body. The sternum 12, as shown, has previously undergone a medical procedure known as median sternotomy. As a result of this procedure, the sternum 12 has been severed, thus permitting a physician access to tissues and/or organs located in the patient's thoracic cavity. However, the sternum 12 has since been reapproximated such that the previously severed portions 12a, 12b are now bound together by the system 10.

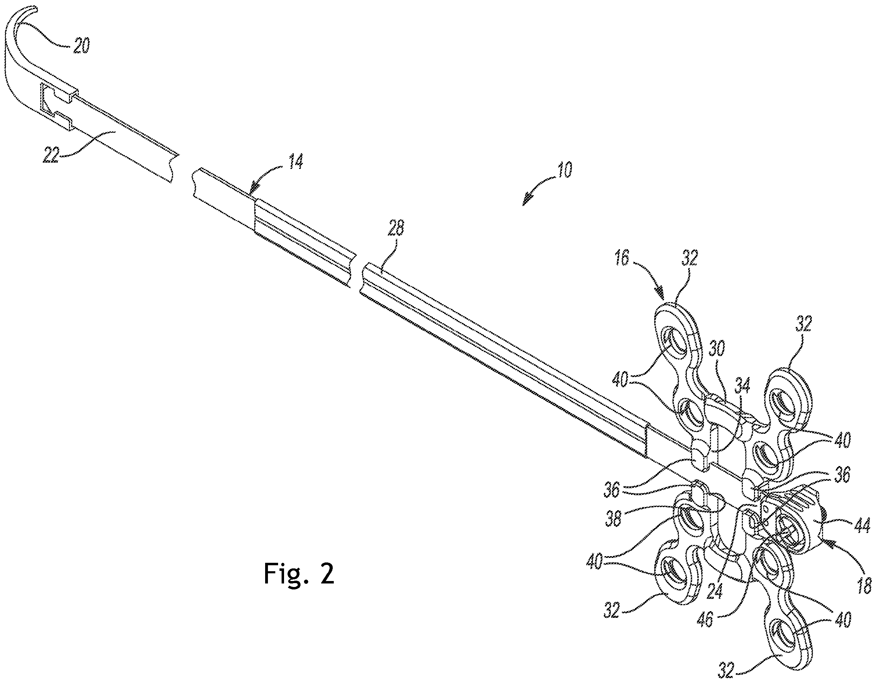

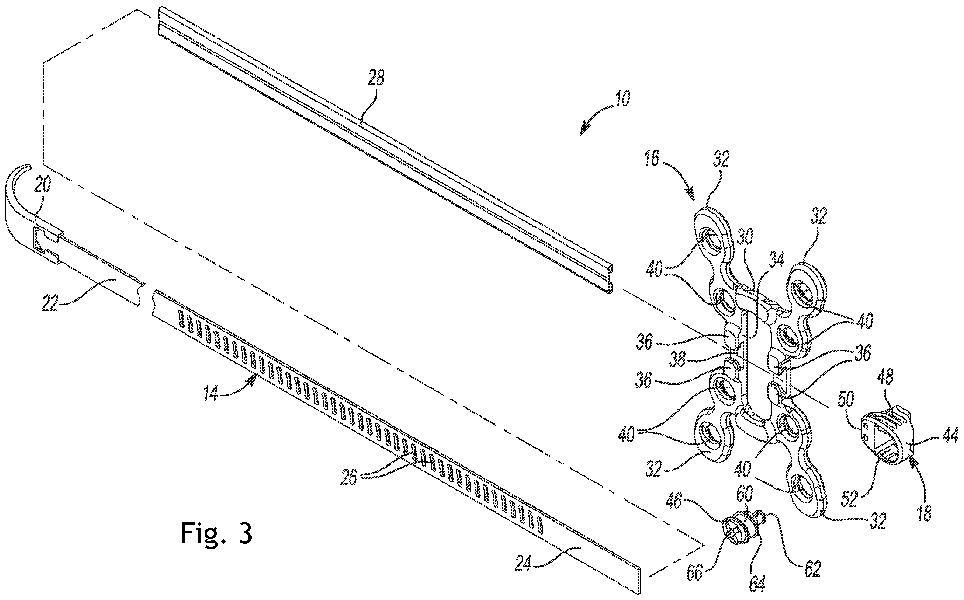

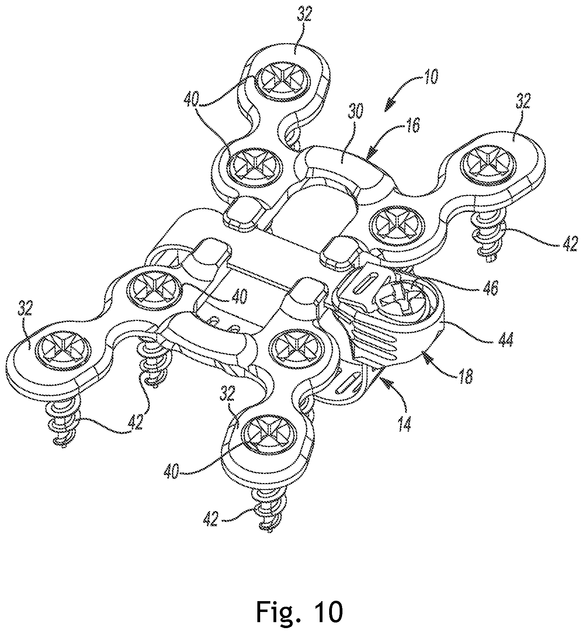

With continued reference to Figures 1-10, the system 10 may include a cerclage or band 14, a bracket 16, and a tensioning device 18. As shown in Figure 2, a needle 20 may be temporarily attached to a first end 22 of the band 14. The needle 20 may be attached to the first end 22 with fasteners and/or a press or snap fit, for example. A second end 24 of the band 14 may be fixed to the tensioning device 18. The band 14 may be a flat, elongated and flexible member formed from a metallic material and/or a polymeric material, for example. In some embodiments, the band 14 could include a braided material. The band 14 may include a plurality of parallel slots 26 formed therein. In some embodiments, a sleeve 28 (Figures 2 and 3) may receive a portion of the band 14. In some embodiments, the sleeve 28 may be molded over the band 14 or otherwise integrally formed therewith.

The bracket 16 may be a plate formed from a metallic and/or polymeric material and may include a body portion 30 and a plurality of legs 32 extending outward from the body portion 30. The bracket 16 may be relatively flexible to enable the bracket 16 to conform to the contours of the sternum 12 when the bracket 16 is fastened thereto (as shown in Figure 1). The body portion 30 may include an opening 34 extending therethrough. The opening 34 may allow the bracket 16 to be readily cut using band cutters, for example, or other standard operating room tools in case the sternum 12 needs to be reopened after installation of the system 10. The body portion 30 may also include a plurality of tabs 36 that cooperate to define a channel 38 through which the band 14 is slidably received. Each of the plurality of legs 32 may include one or more apertures 40 extending therethrough. Self-tapping threaded fasteners 42 may extend through the apertures 40 and may threadably engage the sternum 12 to fix the bracket 16 to the sternum 12 (as shown in Figure 1).

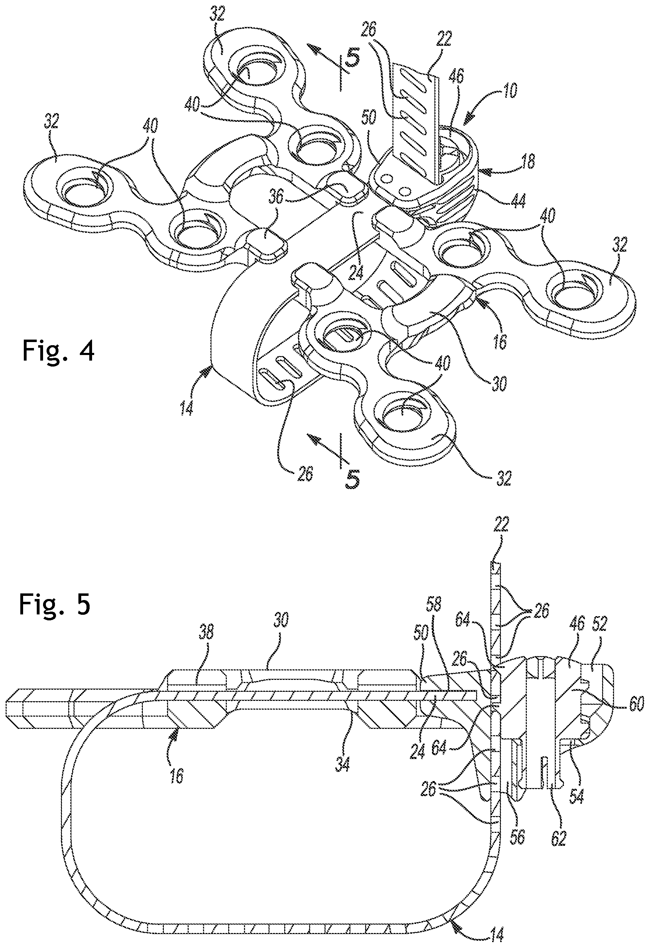

The tensioning device 18 may include a receiver 44 and a tensioning screw 46. The receiver 44 may include a body portion 48 and a tab 50. As shown in Figure 5, the body portion 48 may include a recess 52, a first opening 54 and a second opening 56. The first and second openings 54, 56 may be generally adjacent each other and are in communication with the recess 52. The tab 50 may extend outward from the body portion 48 and may fixedly engage the second end 24 of the band 14. The second end 24 may be received within a slot 58 (Figure 5) in the tab 50 and may be fixed therein by pins, welding, crimping, and/or one or more fasteners, for example, or any other suitable means.

As shown in Figure 5, the tensioning screw 46 may include a head portion 60 and a stem 62. The head portion 60 may include threads 64 and one or more slots 66 that can receive the tip of a screwdriver. The head portion 60 may be received in the recess 52 of the receiver 44 so that the stem 62 extends through the first opening 54 of the receiver 44. As shown in Figure 5, the second end 22 of the band 14 may be inserted through the second opening 56 and the recess 52 of the receiver 44. The threads 64 of the tensioning screw 46 may engage the slots 26 in the band 14 such that when the tensioning screw 46 is rotated relative to the receiver 44, the band 14 is moved up or down (relative to the frame of reference of Figure 5) relative to the receiver 44 based on the direction that the tensioning screw 46 is rotated.

With continued reference to Figures 1-10, a method will be described for attaching the system 10 to the sternum 12 and tensioning the band 14 to reapproximate the sternum 12. With the needle 20 attached to the first end 22 of the band 14 (as shown in Figure 2), the first end 22 of the band 14 may be looped around the posterior side of the sternum 12 so that the band 14 substantially circumscribes the sternum 12. Once the band 14 is looped around end 22 of the band 14 may be inserted up through the receiver 44 (as shown in Figure 5). As shown in Figure 1, the bracket 16 may be positioned so that the fracture separating the two portions 12a, 12b of the sternum 12 is visible in the opening 34 in the bracket 16 (i.e., so that the two of the legs 32 of the bracket 14 are aligned with one portion 12a, 12b of the sternum 12 and the other two legs 32 are aligned with the other portion 12a, 12b of the sternum 12).

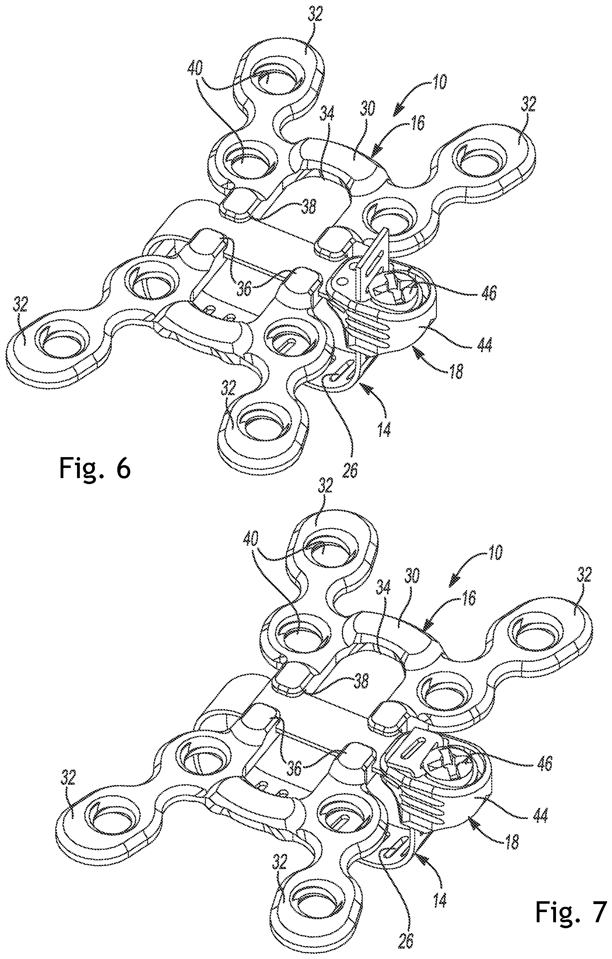

The tensioning device 18 may be used to tighten the band 14 around the sternum 12 to bind the portions 12a, 12b of the sternum 12 together. As described above, the tensioning screw 46 may be rotated relative to the receiver 44 to move the band 14 up (relative to the frame of reference of Figure 5) to tighten the band 14 around the sternum 12 to a desired amount. Once the band 14 has been tightened to the desired amount, the first end 22 of the band 14 may be trimmed (as shown in Figure 6) and subsequently folded over onto the tab 50 of the receiver 44 (as shown in Figure 7).

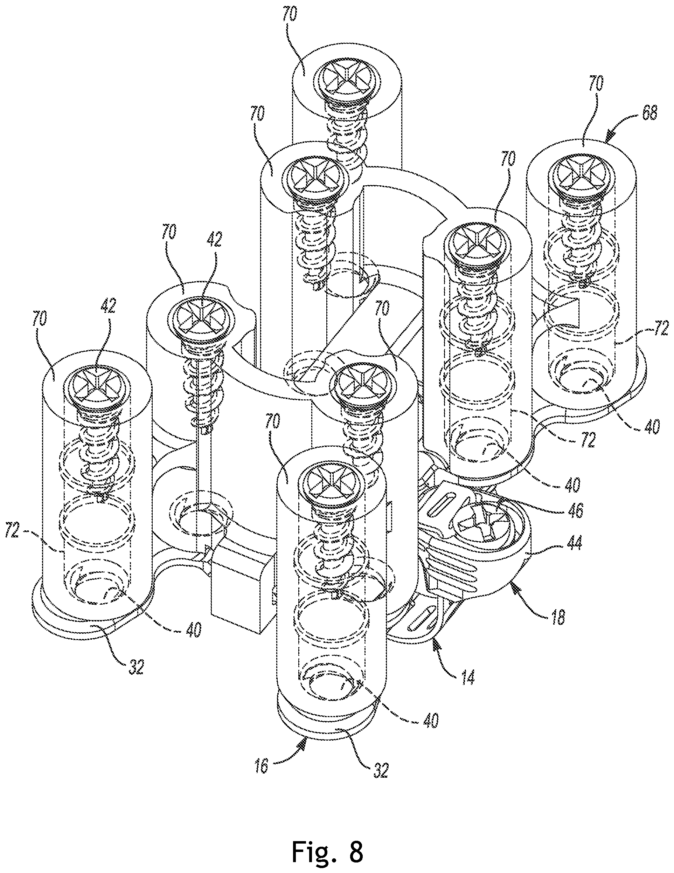

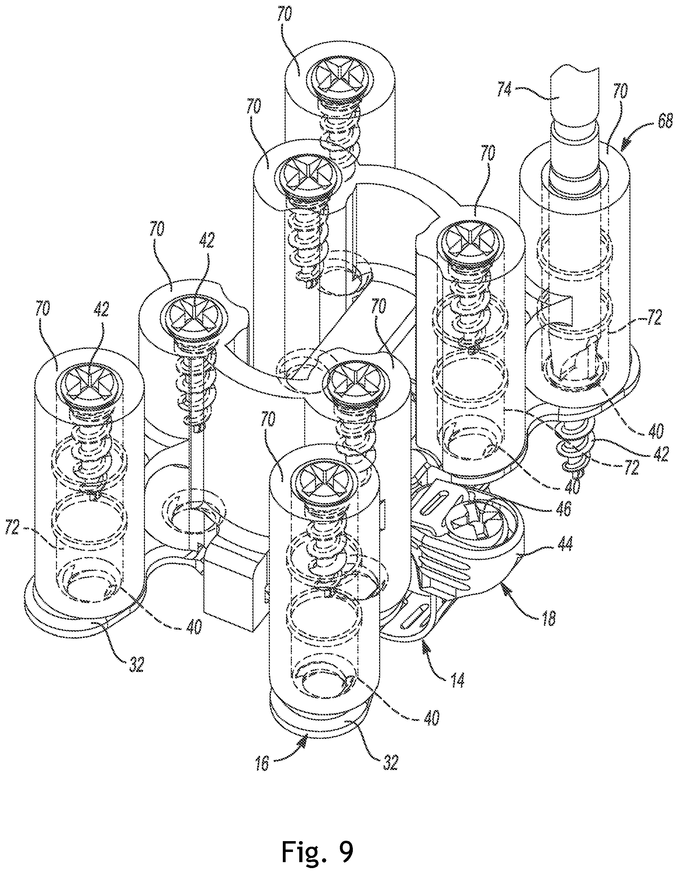

With the band 14 sufficiently tightened around the sternum 12, the bracket 16 may be fixed to the sternum 12 with the fasteners 42. That is, the fasteners 42 may be inserted through the apertures 40 in the bracket 16 and driven into the sternum 12 using a screwdriver (e.g., a manual screwdriver or an electric screwdriver). As shown in Figures 8 and 9, a fixture 68 may be employed to hold the fasteners 42 as they are driven through the apertures 40 and into the sternum 12. The fixture 68 may include a plurality of tubes 70 having apertures 72 sized to receive the fasteners 42. The tubes 70 may have the same spacing and relative orientation as that of the apertures 40 so that the fixture 68 can be aligned relative to the bracket 16 such that the apertures 72 are aligned with the apertures 40 (as shown in Figure 8). With the fixture 68 in this position, a screwdriver 74 (Figure 9) can drive the fasteners through the apertures 72, 40 and into the sternum 12.

While the system 10 is described above as including the bracket 16, it will be appreciated that the band 14 and tensioning device 18 could be used without the bracket 16 to reapproximate the sternum 12.

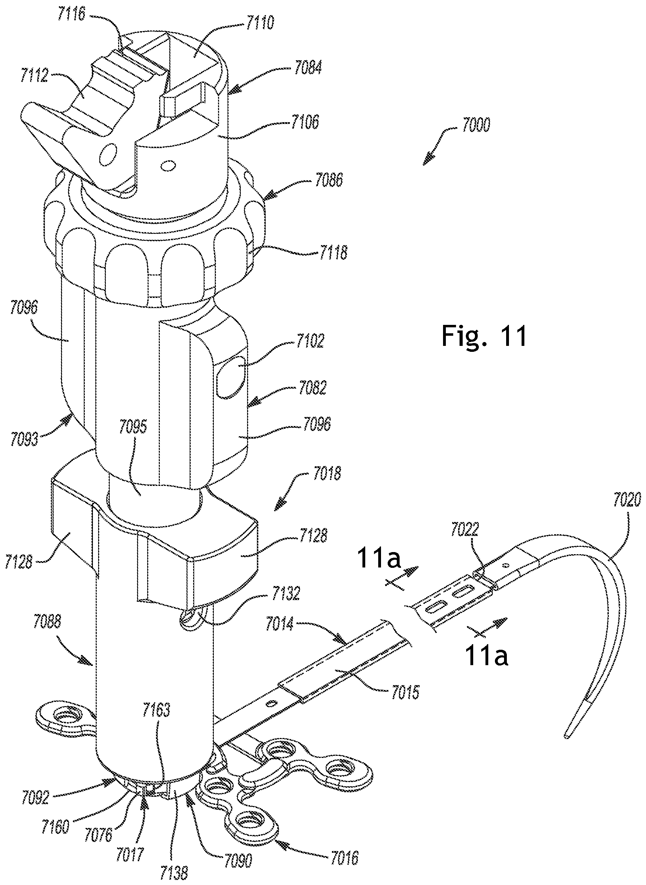

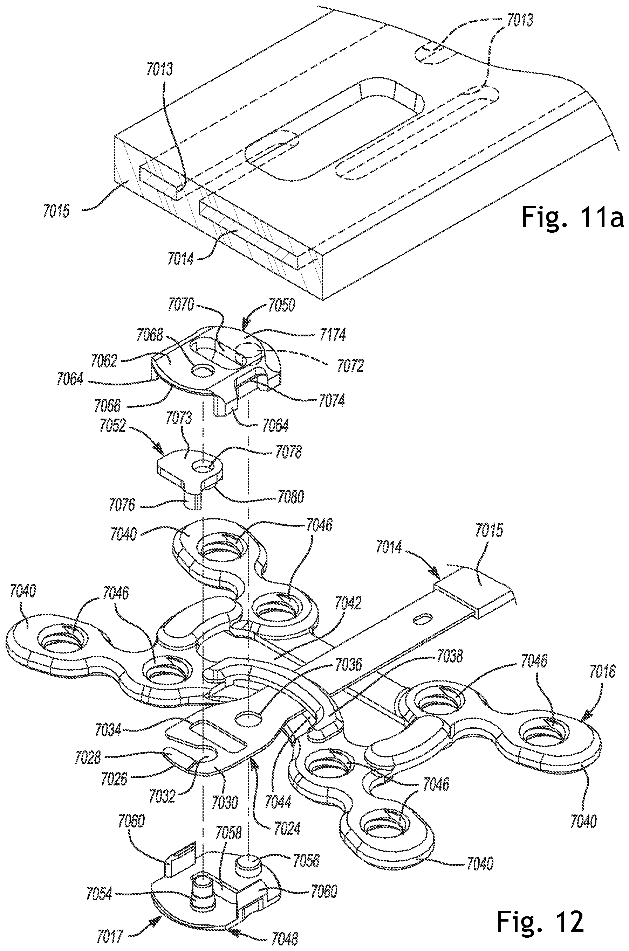

With reference to Figures 11-24, a closure system 7000 according to the invention is provided that may be used to secure portions of a bone (e.g., the sternum 12) to each other. The closure system 7000 may include band 7014, a bracket 7016, a locking device or terminal 7017 and a tensioning device 7018. The band 7014, bracket 7016 and the locking device 7017 may be implantable within the patient's body. As will be described in more detail below, the tensioning device 7018 may operable to tighten the band 7014 around the patient's bone and can be removed from the closure system 7000 prior to completion of the surgical procedure during which the band 7014, bracket 7016 and locking device 7017 are implanted. The closure system 7000 may be an all-inclusive, multi-functional device that can approximate the patient's sternum, tension the band 7014 around the sternum, lock the bank 7014 in the tensioned condition and trim off an excess length of the band 7014 without the assistance of other instruments. A manufacturer can provide the closure system 7000 to a healthcare facility and/or healthcare personnel with the tensioning device 7018 pre-attached to the locking device 7017 (which the manufacturer may pre-attach to the band 7014 and bracket 7016).

The band 7014 may be a flat, elongated and flexible member formed from a metallic material and/or a polymeric material, for example. In some embodiments, the band 7014 could include a braided material. In some embodiments, the band 7014 could include a sleeve 7015 covering some or all of the band 7014. For example, as shown in Figure 11a, the sleeve 7015 may be a molded polymer and may be molded over the band 7014. The sleeve 7015 may be molded through apertures 7013 in the band 7014 to hinder or prevent delamination or separation of the sleeve 7015 from the band 7014 when the band 7014 is sliding through the locking device 7017 or locked in place by the locking device 7017. As shown in Figure 11, a needle 7020 may be temporarily attached to a first end 7022 of the band 7014. The needle 7020 may be attached to the first end 7022 with fasteners and/or a press or snap fit, for example.

A second end 7024 of the band 7014 may be fixed to the locking device 7017. As shown in Figure 12, the second end 7024 of the band 7014 may include a generally hook-shaped, resiliently flexible tab 7026. A distal end 7028 of the tab 7026 may be inclined or angled relative to a proximal end 7030 of the tab 7026. The tab 7026 partially defines a first aperture 7032. The second end 7024 may also include a second aperture 7034 and a third aperture 7036. The second aperture 7034 can be an elongated, generally rectangular opening sized to allow the first end 7022 of the band 7014 to pass therethrough.

As shown in Figure 12, the bracket 7016 may be a plate formed from a metallic and/or polymeric material and may include a body portion 7038 and a plurality of legs 7040 extending outward from the body portion 7038. The bracket 7016 may be relatively flexible to enable the bracket 7016 to conform to the contours of the sternum 12 when the bracket 7016 is fastened thereto. The body portion 7038 may include an opening 7042 extending therethrough. The opening 7042 may allow the bracket 7016 to be readily cut using band cutters, for example, or other standard operating room tools in case the portions 12a, 12b of the sternum 12 need to be separated to open the patient's chest cavity after installation of the closure system 7000. The body portion 7038 may also include a channel 7044 through which the band 7014 is slidably received. Each of the plurality of legs 7040 may include one or more threaded apertures 7046 extending therethrough. Threaded fasteners (not shown) may extend through the apertures 7046 and may threadably engage the sternum 12 to fix the bracket 7016 to the sternum 12. The shape of the body portion 7038, the shape and size of the channel 7044 relative to the band 7014 and the manner in which the bracket 7016 is retained by the band 7014 and the locking device 7017 allows for substantial adjustability of the bracket 7016 relative to the sternotomy line of the patient's sternum 12 before the bracket 7016 is fixed to the sternum 12 with the fasteners.

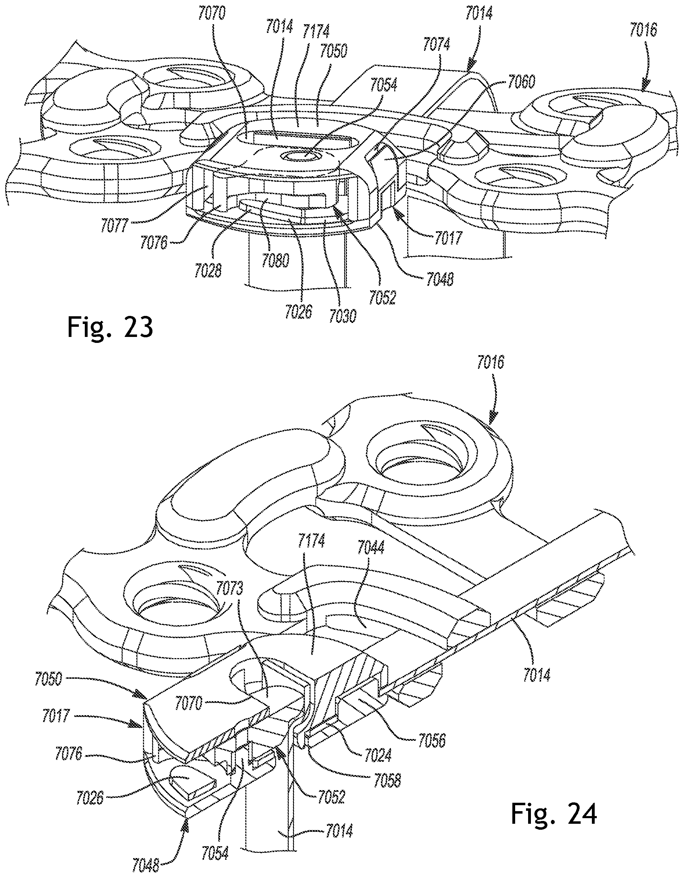

As shown in Figure 12, the locking device 7017 may include a first casing member 7048, a second casing member 7050 and a cam member 7052. The first casing member 7048 may include a first protrusion 7054, a second protrusion 7056, an elongated aperture 7058, and a pair of resiliently flexible arms 7060. The second casing member 7050 may include a main body 7062 and a pair of arms 7064 that cooperate to form a recess 7066. The main body 7062 may include a first aperture 7068, an elongated second aperture 7070 and a third aperture 7072. The cam member may include a body 7073 and a tab 7076 extending therefrom. The body 7073 may include an aperture 7078. As shown in Figure 23, a recess 7080 may be formed in a portion of the body 7073 and the tab 7076.

The arms 7060 of the first casing member 7048 may snap into engagement with recesses 7074 in the arms 7064 of the second casing member 7050 so that the first and second casing members 7048, 7050 cooperate to define a cavity 7077 (Figure 23) therebetween in which the cam member 7052 and the second end 7024 of the band 7014 are disposed. The first protrusion 7054 of the first casing member 7048 may extend through the first aperture 7032 of the second end 7024 of the band 7014, through the aperture 7078 in the cam member 7052 and into the first aperture 7068 of the second casing member 7050. As shown in Figure 24, the elongated apertures 7058, 7034, 7070 may be aligned with each other so that the first end 7022 of the band 7014 can pass therethrough. The second protrusion 7056 of the first casing member 7048 may be received in the third aperture 7036 of the band 7014 and in the third aperture 7072 of the second casing member 7050.

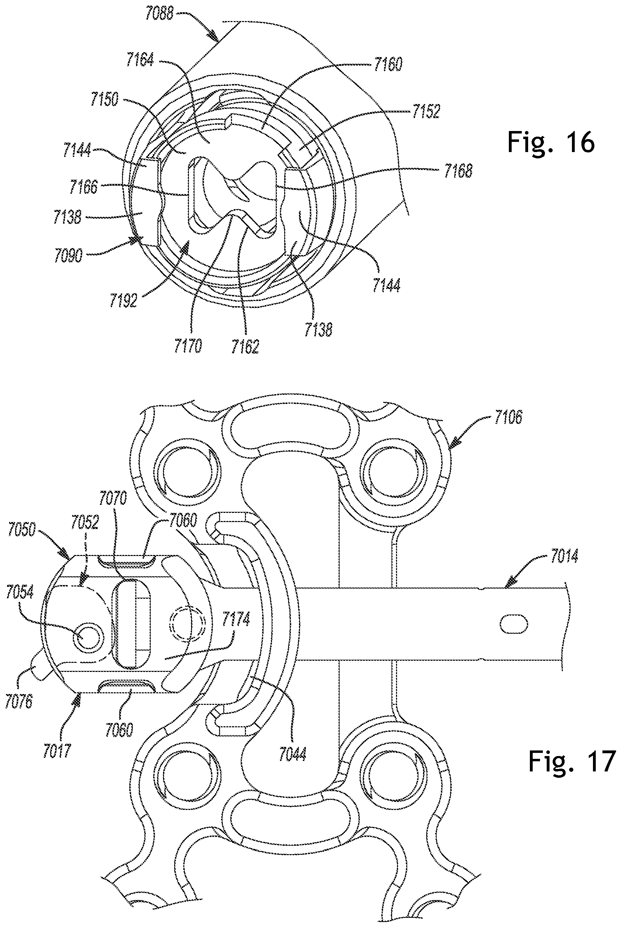

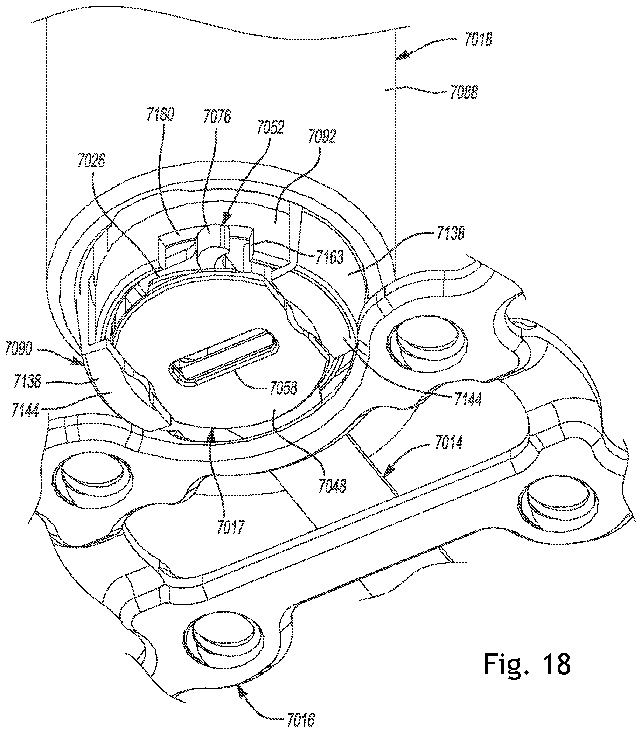

The cam member 7052 may be rotatable relative to the first and second casing members 7048, 7050 and the band 7014 about a longitudinal axis of the first protrusion 7054 between an unlocked position (Figures 17 and 18) and a locked position (Figures 23 and 24). In the unlocked position, the body 7073 of the cam member 7052 is positioned to allow the band 7014 to freely pass through the elongated apertures 7058, 7034, 7070. In the locked position, the body 7073 of the cam member 7052 clamps the band 7014 against the second casing member 7050 (as shown in Figure 24) to restrict or prevent movement of the band 7014 through the elongated apertures 7058, 7034, 7070. As shown in Figure 24, in the locked position, the cam member 7052 bends the band 7014 into an S-shape, which further restricts movement of the band 7014 relative to the locking device 7017. As shown in Figure 23, the distal end 7028 of the tab 7026 of the second end 7024 of the band 7014 restricts or prevents rotation of the cam member 7052 from the locked position toward the unlocked position, while allowing the cam member 7052 to rotate from the unlocked position to the locked position.

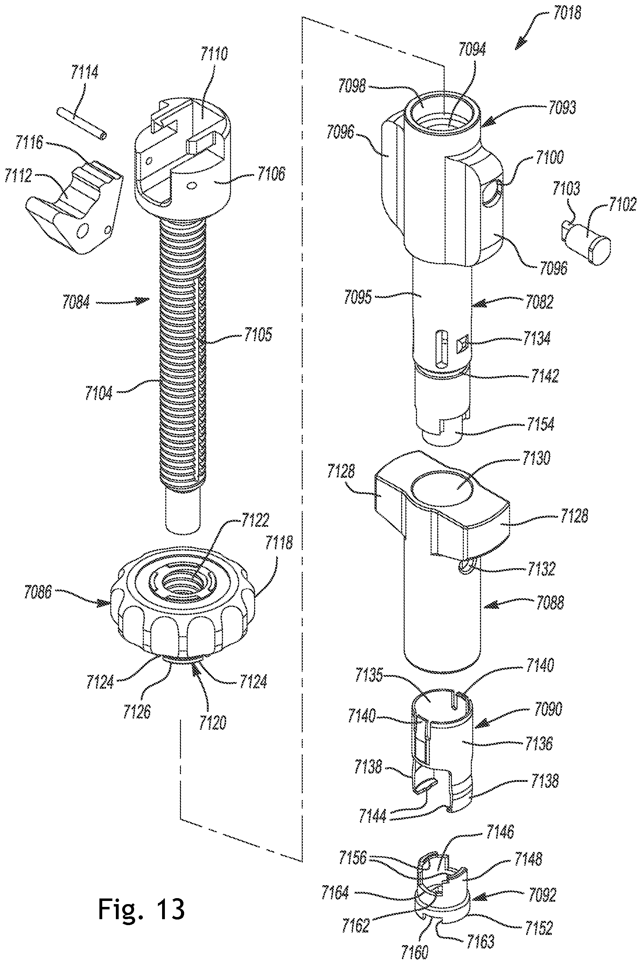

Referring now to Figures 13-15, the tensioning device 7018 may include a main body 7082, a tensioning rod 7084, an adjustment member 7086, a release sleeve 7088, a retaining collar 7090 and a trimming member 7092. The main body 7082 may be a generally cylindrical member including a gripping portion 7093 and a shaft portion 7095. An unthreaded central aperture 7094 extends entirely through the gripping portion 7093 and the shaft portion 7095. The gripping portion 7093 may include a pair of flanges 7096 extending radially outwardly therefrom. An axial end of the gripping portion 7093 may include a recess 7098 in communication with the aperture 7094. A radially extending aperture 7100 may extend through one of the flanges 7096 and communicate with the central aperture 7094. An anti-rotation pin 7102 may be received in the aperture 7100 and may engage the tensioning rod 7084.

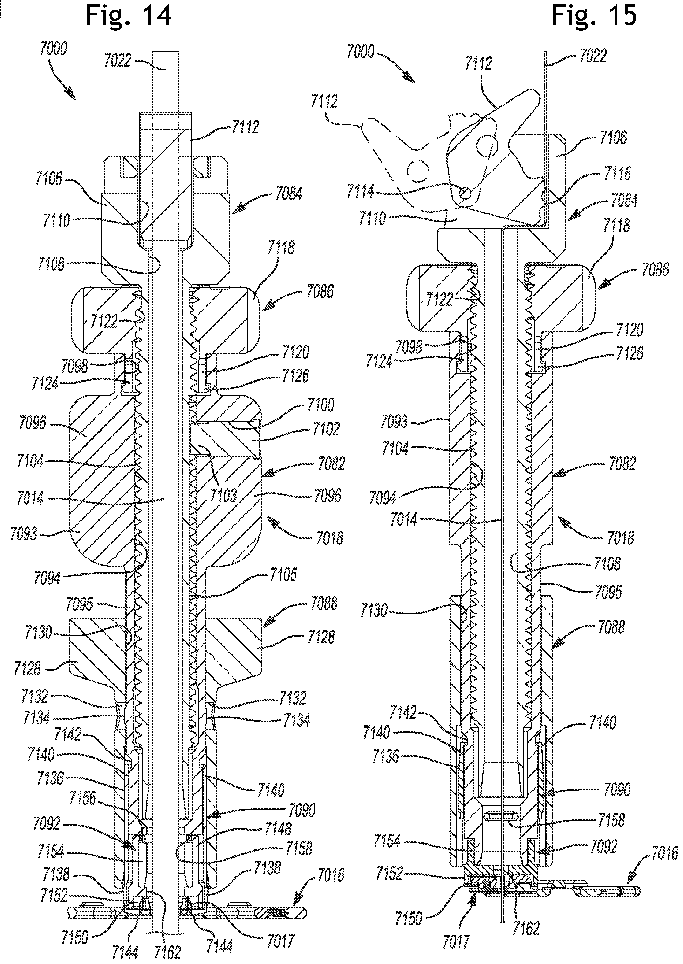

The tensioning rod 7084 may include a threaded shaft 7104 and a head 7106. The threaded shaft 7104 may be movably received in the unthreaded central aperture 7094 of the main body 7082. A tip 7103 of the anti-rotation pin 7102 is received in a slot 7105 of the threaded shaft 7104 to prevent rotation of the tensioning rod 7084 relative to the main body 7082 while still allowing the tensioning rod 7084 to move in an axial direction (a direction along a longitudinal axis of the shaft 7104) relative to the main body 7082. The tensioning rod 7084 includes a central aperture 7108 that extends entirely through the threaded shaft 7104 and the head 7106. As shown in Figures 14 and 15, the band 7014 may extend through the central aperture 7108 of the tensioning rod 7084. The head 7106 includes a larger diameter than the threaded shaft 7104 and includes a recess 7110 in communication with the central aperture 7108.

A toggle lock member 7112 may be rotatably received in the recess 7110. A pin 7114 (Figure 13) may be attached to the head 7106 and rotatably support the toggle lock member 7112 for movement between an unlocked position (shown in phantom lines in Figure 15) and a locked position (shown in solid lines in Figure 15). In the unlocked position, the toggle lock member 7112 allows the band 7014 to freely move relative to the tensioning rod 7084 through the central aperture 7108. In the locked position, a clamping portion 7116 of the toggle lock member 7112 clamps the band 7014 against the head 7106 to restrict or prevent movement of the first end 7022 of the band 7014 relative to the tensioning rod 7084.

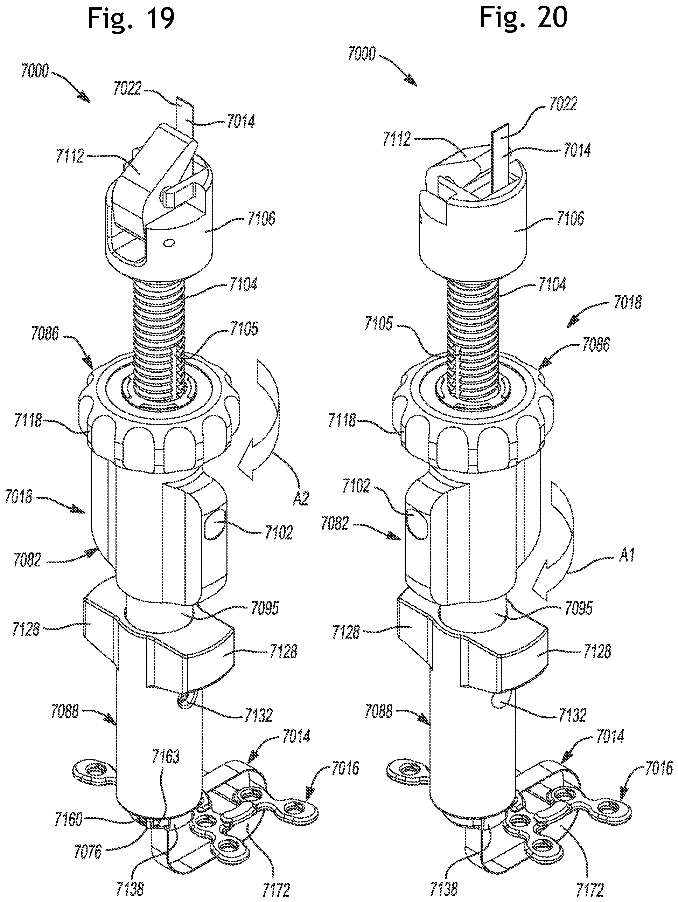

The adjustment member 7086 may include a gripping wheel 7118 and a neck 7120. A central aperture 7122 extends through the gripping wheel 7118 and the neck 7120. At least a portion of the central aperture 7122 that extends through the gripping wheel 7118 is threaded and threadably engages the threaded shaft 7104 of the tensioning rod 7084. The neck 7120 is received in the recess 7098 of the main body 7082 and may include a plurality of resiliently flexible arms 7124 having barbed tips 7126 that snap into engagement with the main body 7082. In this manner, the adjustment member 7086 is axially fixed to the main body 7082, yet rotatable relative to the main body 7082. Accordingly, rotation of the adjustment member 7086 relative to the main body 7082 causes corresponding axial movement of the tensioning rod 7084 relative to the main body 7082 between a first position (shown in Figures 14 and 15) and a second position (shown in Figures 19 and 20).

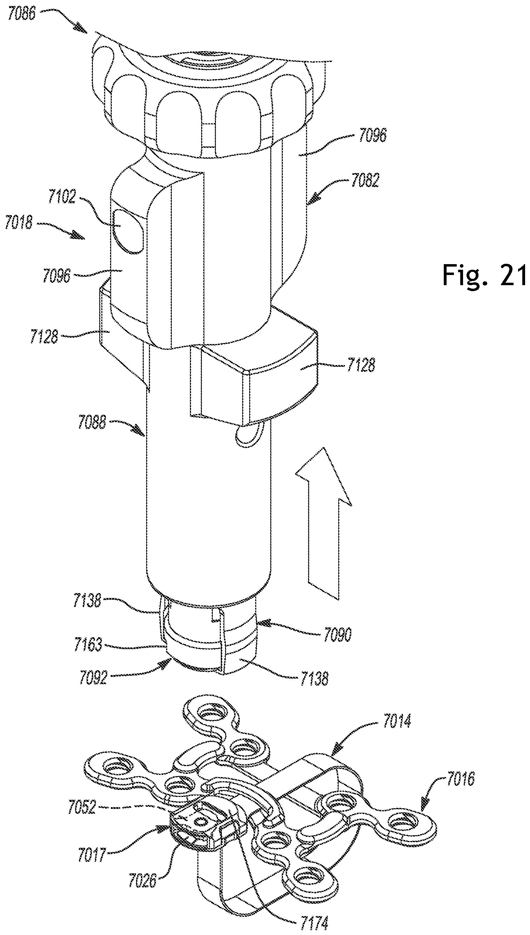

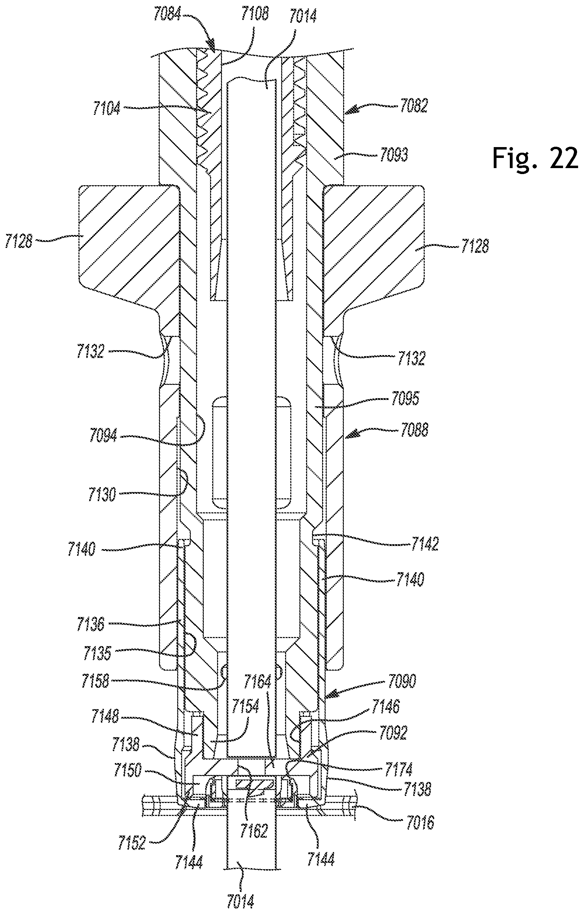

The release sleeve 7088 may be a generally tubular body including a pair of flanges 7128 extending radially outward. A central aperture 7130 may extend axially through the release sleeve 7088. The shaft portion 7095 of the main body 7082 may be movably received in the central aperture 7130. The release sleeve 7088 may also include a pair of radially extending apertures 7132 in communication with the central aperture 7130. Protrusions 7134 (Figure 14) extending radially outwardly from the shaft portion 7095 of the main body 7082 may releasably engage the radially extending apertures 7132 to releasably retain the release sleeve 7088 relative to the main body 7082 in a first position (shown in Figures 14, 15, 19 and 20). Application of a sufficiently strong axially upward force on the release sleeve 7088 relative to the main body 7082 can snap the protrusions 7134 out of the apertures 7132 to allow the release sleeve 7088 to move axially upward relative to the main body 7082 to a second position (shown in Figures 21 and 22).

The retaining collar 7090 may include a generally tubular body 7136 and a pair of retaining arms 7138 extending axially downward from the tubular body 7136. The tubular body 7136 may include a central aperture 7135 extending axially therethrough. The tubular body 7136 may also include a plurality of flexible barbed arms 7140 that snap into engagement with an annular groove 7142 formed in the shaft portion 7095 of the main body 7082. The retaining arms 7138 may be resiliently flexible relative to the tubular body 7136. As shown in Figure 18, radially inwardly extending tabs 7144 of the retaining arms 7138 may releasably engage the locking device 7017 to releasably secure the tensioning device 7018 thereto.

As shown in Figure 13, the trimming member 7092 may be a generally cylindrical member including a first recess 7146 formed in a first end portion 7148 and a second recess 7150 (Figures 14 and 15) formed in a second end portion 7152. A distal end 7154 of the shaft portion 7095 of the main body 7082 may be received in the first recess 7146. Barbed fingers 7156 (Figure 13) formed on the first end portion 7148 may fixedly engage one or more grooves or apertures 7158 (Figures 14 and 15) via a snap fit.

When the retaining collar 7090 is engaged with the locking device 7017, the locking device 7017 may be received in the second recess 7150 (as shown in Figures 14, 15, 18 and 22). The second end portion 7152 may include a notch 7160 through which the tab 7076 of the cam member 7052 extends when the cam member 7052 is in the unlocked position (as shown in Figure 18). When the main body 7082 is rotated (which causes the trimming member 7092 to rotate with the main body 7082) relative to the locking device 7017 in the direction indicated by arrow A1 in Figure 20, a surface 7163 (shown best in Figure 18) defining the notch 7160 pushes the tab 7076, thereby causing the cam member 7052 to rotate into the locked position.

A bowtie-shaped aperture 7162 (Figure 16) may be formed through a wall 7164 separating the first and second recesses 7146, 7150. The aperture 7162 may include first and second relatively wide end portions 7166, 7168 and a relatively narrow central portion 7170 disposed between the end portions 7166, 7168. It will be appreciated that, in some embodiments, the aperture 7162 could be alternatively shaped. When the retaining collar 7090 is engaged with the locking device 7017, the aperture 7162 is aligned with the aperture 7070 of the locking device 7017 so that the band 7014 can pass through the aperture 7162 and extend into the central aperture 7094 of the main body 7082 (as shown in Figures 14 and 15). In some embodiments, one or more blades (not shown) may extend radially into the central portion 7170 of the aperture 7162. When the main body 7082 is rotated (which causes the trimming member 7092 to rotate with the main body 7082) relative to the locking device 7017, one or more surfaces defining the aperture 7162 may cut or shear the band 7014 to the length shown in Figures 21 and 23.

With continued reference to Figures 11-24, operation of the closure system 7000 will be described in detail. As described above, the bracket 7016 and the locking device 7017 may cooperate to secure the band 7014 around portions 12a, 12b of a patient's bone 12. The tensioning device 7018 may tighten the band 7014 around the bone 12 and trim off excess length of the band 7014.

As shown in Figure 11, the needle 7020 may be attached to the first end 7022 of the band 7014 to aid the surgeon in passing the first end 7022 of the band 7014 through tissue surrounding the bone 12. Thereafter, the surgeon may remove the needle 7020. With the cam member 7052 in the unlocked position, the first end 7022 of the band 7014 may be passed through the apertures 7058, 7034, 7070 of the locking device 7017. Thereafter, with the tensioning device 7018 attached to the locking device 7017 (as shown in Figure 11), the first end 7022 of the band 7014 may be passed up through the aperture 7162 in the trimming member 7092, into the central aperture 7094 of the main body 7082 and through the central aperture 7108 of the tensioning rod 7084, as shown in Figures 14 and 15.

With the first end 7022 of the band 7014 extending through the head 7106 of the tensioning rod 7084, the toggle lock member 7112 may be moved from the unlocked position (shown in phantom lines in Figure 15) to the locked position (shown in solid lines in Figure 15) to clamp the first end 7022 of the band 7014 in place and restrict movement of the first end 7022 of the band 7014 relative to the tensioning rod 7084.

With the toggle lock member 7112 in the locked position, the surgeon may grip the gripping portion 7093 of the main body 7082 with one hand and may grip the gripping wheel 7118 of the adjustment member 7086 with the other hand and rotate the adjustment member 7086 relative to the main body 7082 in the direction A2 (Figure 19) while holding the main body 7082 stationary relative to the locking device 7017. As described above, such rotation of the adjustment member 7086 relative to the main body 7082 causes the tensioning rod 7084 to move axially (i.e., in a direction along the longitudinal axis of the tensioning rod 7084) upward relative to the adjustment member 7086, the main body 7082 and the locking device 7017, as shown in Figure 19. As described above, engagement between the anti-rotation pin 7102 and the slot 7105 prevents relative rotation between the tensioning rod 7084 and the main body 7082 while the adjustment member 7086 rotates relative to the main body 7082. Upward axial movement of the tensioning rod 7084 relative to the main body 7082 with the first end 7022 of the band 7014 clamped in place with the toggle lock member 7112 moves the first end 7022 upward relative to the locking device 7017, thereby tightening the band 7014 around the patient's bone 12 (i.e., the circumference of a looped portion 7172 (Figure 19) of the band 7014 is reduced). Rotation of the adjustment member 7086 relative to the main body 7082 in a direction opposite the direction A2 causes the tensioning rod 7084 to move axially downward relative to the adjustment member 7086, the main body 7082 and the locking device 7017, thereby loosening the looped portion 7172 of the band 7014.

Once the surgeon has rotated the adjustment member 7086 to achieve a desired tension in the band 7014, the surgeon may grip the release sleeve 7088 with one hand and grip the gripping portion 7093 of the main body 7082 with the other hand and rotate the main body 7082 relative to the locking device 7017, the release sleeve 7088 and the retaining collar 7090 in the direction A1, as shown in Figure 20. As described above, rotating the main body 7082 in the direction A1 causes rotation of the trimming member 7092 in the direction A1 relative to the locking device 7017, which causes the surface 7163 of the notch 7160 to push the tab 7076 of the cam member 7052 into the locked position (Figure 23). As described above, when the cam member 7052 is in the locked position, the cam member 7052 clamps the band 7014 relative to the second casing member 7050 of the locking device 7017 to restrict or prevent relative movement of the band 7014 relative to the locking device 7017. In some embodiments, the cam member 7052 can be moved from the unlocked position to the locked position by rotating the main body 7082 approximately ninety degrees relative to the locking device 7017. It will be appreciated that, in some embodiments, more or less than ninety degrees of rotation of the main body 7082 may be required to move the cam member 7052 from the unlocked position to the locked position.

After moving the cam member 7052 to the locked position, the surgeon may continue to rotate the main body 7082 relative to the locking device 7017, the release sleeve 7088 and the retaining collar 7090 in the direction A1 to trim the band 7014 at a location substantially flush with an upper surface 7174 of the second casing member 7050 of the locking device 7017 (as shown in Figures 23 and 24) or at a location between the upper surface 7174 and the distal end 7154 of the main body 7082. This continued rotation of the main body 7082 and the trimming member 7092 relative to the locking device 7017 causes surfaces defining the aperture 7162 of the trimming member 7092 to cut or shear the band 7014. In some embodiments, the band 7014 may be cut by the trimming member 7092 within two full rotations (720 degrees) of the main body 7082 after the cam member 7052 is rotated to the locked position. It will be appreciated that, in some embodiments, more or less than 720 degrees of rotation of the main body 7082 and trimming member 7092 may be required to trim the band 7014.

After the band 7014 has been trimmed, the surgeon may slide the release sleeve 7088 axially upward relative to the main body 7082, as shown in Figures 21 and 22. With the release sleeve 7088 in this upward position, the retaining arms 7138 are free to flex laterally outward away from the locking device 7017 so that the retaining arms 7138 can be disengaged from the locking device 7017, thereby disengaging the tensioning device 7018 from the locking device 7017, as shown in Figure 21. After removing the tensioning device 7018 from the locking device 7017, the tensioning device 7018 can be discarded (i.e., placed in a waste receptacle) or sterilized and reused with a different band 7014 and locking device 7017.

Threaded fasteners (not shown) may be driven through the apertures 7046 of the bracket 7016 and into the patient's bone 12 to further secure the bracket 7016 relative to the bone 12. For example, this can be done after the tensioning device 7018 is disengaged from the locking device 7017 or any time after the band 7014 is tightened around the bone 12. The band 7014, the locking device 7017, the bracket 7016 and the threaded fasteners are made from implantable materials and can be left inside the patient's body after the surgical procedure is completed. Once the patient's bone 12 heals the band 7014, the locking device 7017, the bracket 7016 and the threaded fasteners may be removed from the patient's body in a subsequent surgical procedure.

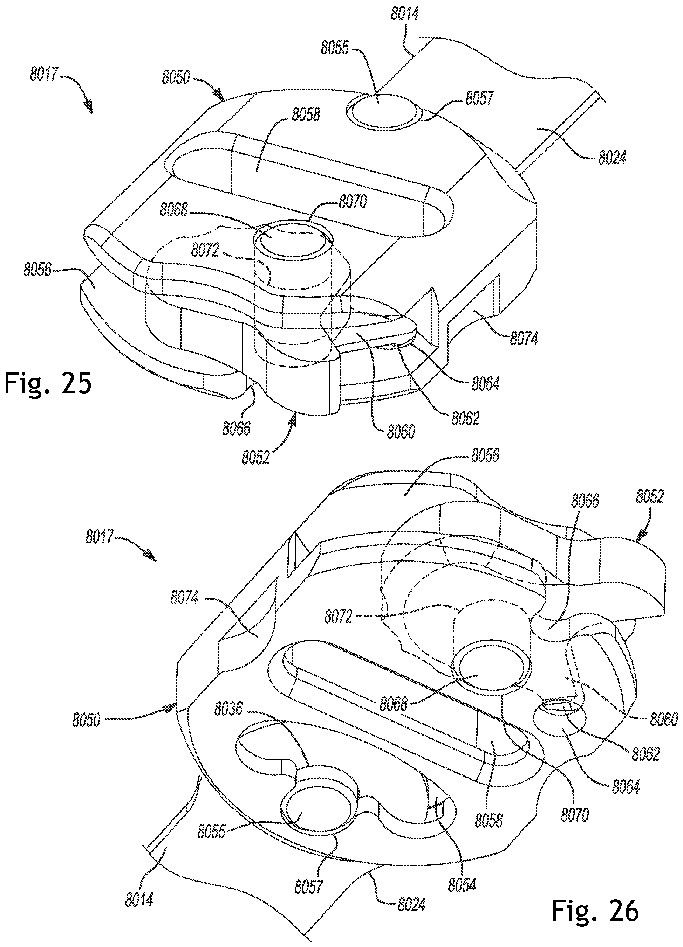

With reference to Figures 25-28, another locking device 8017 and band 8014 are provided that can be incorporated into the closure system 7000 instead of the locking device 7017 and band 7014. The structure and function of the locking device 8017 and band 8014 may be similar or identical to that of the locking device 7017 and band 7014 described above, apart from exceptions noted herein and/or shown in the figures. Therefore, similar features will not be described again in detail.

The locking device 8017 may include a casing 8050 and a cam member 8052. The casing 8050 may be a one-piece, unitary body having a first cavity 8054 (Figure 26), a second cavity 8056 and an elongated aperture 8058. A first end 8024 of the band 8014 may be received in the first cavity 8054. A pin 8055 or other fastener may be received in apertures 8057 of the casing and an aperture 8036 in the first end 8024 of the band 8014 to securely connect the first end 8024 to the casing 8052 (it will be appreciated that the first end 8024 of the band 8014 may not include an aperture like the aperture 7034 of the band 7014 or a tab like the tab 7026 of the band 7014).

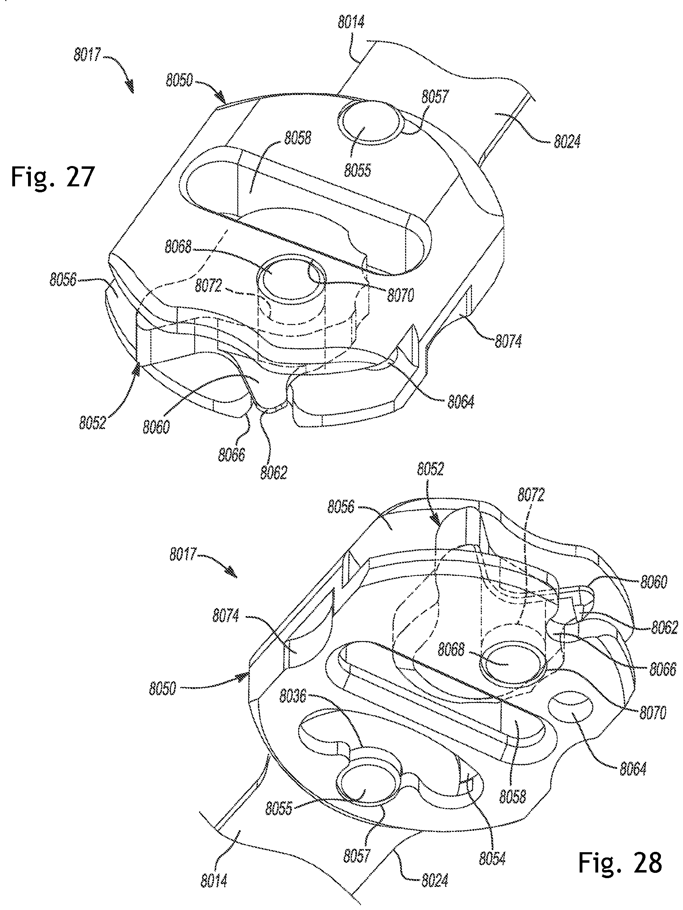

The cam member 8052 may be received within the second cavity 8056 and may be rotatable relative to the casing 8050 between an unlocked position (Figures 25 and 26) and a locked position (Figures 27 and 28). The cam member 8052 may be similar or identical to the cam member 7052, except the cam member 8052 may include a deflectable tab 8060 extending outwardly therefrom. The tab 8060 may include a barbed tip 8062 that releasably engages a first aperture 8064 in the casing 8050 in the unlocked position and releasably engages a second aperture 8066 in the casing 8050 in the locked position. The first and second apertures 8064, 8066 could be holes, notches, recesses, slots or another other opening. A pin 8068 or other fastener may extend through apertures 8070 in the casing 8050 and an aperture 8072 in the cam member 8052 to rotatably attach the cam member 8052 to the casing 8050. The tabs 7144 (Figure 18) of retaining collar 7090 may engage recesses 8074 formed in the casing member 8050 to releasably secure the locking device 8017 to the tensioning device 7018. While not shown in Figures 27 and 28, in the locked position, the cam member 8052 may clamp a portion (not shown) of the band 8014 within the elongated aperture 8058 of the casing 8050, thereby fixing the band 8014 relative to the casing 8050 in the manner described above with respect to the locking device 7017 and band 7014.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A closure system (7000) for securing a first bone portion (12a) to a second bone portion (12b), the closure system comprising:
a band (7014) adapted to be looped around the first and second bone portions(12a, 12b);
a locking terminal (7017) fixedly engaging a first portion (7024) of the band and selectively fixedly engaging a second portion (7022) of the band; and
a tensioning device (7018) including a body (7082) and a threaded rod (7084), the threaded rod (7084) received in the body (7082) and engaging the band (7014),
**characterised in that** movement of the threaded rod relative to the body (7082) in a direction along the longitudinal axis of the rod moves the second portion (7022) of the band relative to the first portion (7024) of the band in the direction along the longitudinal axis of the rod.

2. The closure system (7000) of Claim 1, wherein the tensioning device (7018) includes a sleeve (7088) that slidably receives the body (7082).

3. The closure system (7000) of Claim 2, wherein the tensioning device (7018) includes a resiliently flexible collar (7090) received in the sleeve (7088) and receiving a portion of the body (7082).

4. The closure system (7000) of Claim 3, wherein the collar (7090) includes a first axial end engaging an annular groove in the body and a second axial end that removably engages the locking terminal.

5. The closure system (7000) of Claim 4, wherein the sleeve (7088) is axially movable relative to the body (7082) and the collar (7090) between a first position restricting radial flexing of the collar (7090) and a second position allowing the collar to flex radially outward relative to the body(7082).

6. The closure system (7000) of Claim 1, wherein the tensioning device (7018) includes an adjustment member (7086) partially received in the body (7082) and threadably engaging the threaded rod (7084), the adjustment member (7086) is rotatable relative to the body (7082) and is axially fixed relative to the body (7082) such that rotation of the adjustment member (7086) relative to the body (7082) causes corresponding axial movement of the threaded rod (7084) relative to the body (7082).

7. The closure system (7000) of Claim 6, wherein the tensioning device (7018) includes an anti-rotation pin (7102) extending through the body (7082) and engaging the threaded rod (7084) to restrict relative rotation between the threaded rod (7084) and the body (7082) while allowing relative axial movement between the threaded rod (7084) and the body (7082).

8. The closure system (7000) of Claim 1, wherein the tensioning device (7018) includes a toggle lock (7112) mounted to the threaded rod (7084) and movable relative to the threaded rod (7084) between an unlocked position allowing the band (7014) to move through an aperture in the threaded rod (7084) and a locked position in which the toggle lock engages the band (7014) and restricts movement of the band (7104) relative to the threaded rod (7084).

9. The closure system (7000) of Claim 1, wherein the locking terminal (7017) includes a casing (7048) and a cam member (7052), the casing engaging the first portion of the band (7024), the cam member (7052) is rotatable relative to the casing and the first portion of the band (7024) between an unlocked position allowing the second portion of the band (7022) to move through the casing (7084) and a locked position in which the cam member (7052) engages the second portion of the band to restrict movement of the second portion relative to the casing (7084) and the first portion (7024).

10. The closure system (7000) of Claim 9, wherein the casing (7048) includes a first casing member (7048) and a second casing member (7050), the first portion of the band (7024) and the cam member (7052) are disposed between the first and second casing members (7048, 7050).

11. The closure system (7000) of Claim 10, wherein the first and second casing members (7048, 7050) and the first portion of the band (7024) all include apertures (7058, 7034, 7070) that are aligned with each other to allow the second portion of the band (7022) to pass therethrough.

12. The closure system (7000) of Claim 9, wherein the casing is a one-piece, unitary body.

13. The closure system (7000) of Claim 9, wherein the cam member (7052) clamps the second portion (7022) of the band (7014) against the casing in the locked position.

14. The closure system (7000) of Claim 9, wherein the first portion of the band (7024) includes a deflectable tab (7026) that allows movement of the cam member (7052) from the unlocked position to the locked position and restricts movement of the cam member (7052) from the locked position to the unlocked position.

15. The closure system (7000) of Claim 9, wherein the cam member (7052) includes a deflectable tab that releasably engages the casing in the locked position to restrict the cam member (7052) from moving out of the locked position.

## Patentansprüche

1. Verschlusssystem (7000) zum Sichern eines ersten Knochenabschnitts (12a) an einem zweiten Knochenabschnitt (12b), wobei das Verschlusssystem Folgendes beinhaltet:
ein Band (7014), das angepasst ist, um um den ersten und zweiten Knochenabschnitt (12a, 12b) herum geschlungen zu werden;
eine Verriegelungsklemme (7017), die einen ersten Abschnitt (7024) des Bands fest in Eingriff nimmt und einen zweiten Abschnitt (7022) des Bands selektiv fest in Eingriff nimmt;
und eine Spannvorrichtung (7018), die einen Körper (7082) und einen Gewindestab (7084) umfasst, wobei der Gewindestab (7084) in dem Körper (7082) aufgenommen wird und das Band (7014) in Eingriff nimmt,
**dadurch gekennzeichnet, dass** eine Bewegung des Gewindestabs relativ zu dem Körper (7082) in einer Richtung entlang der Längsachse des Stabs den zweiten Abschnitt (7022) des Bands relativ zu dem ersten Abschnitt (7024) des Bands in der Richtung entlang der Längsachse des Stabs bewegt.

2. Verschlusssystem (7000) gemäß Anspruch 1, wobei die Spannvorrichtung (7018) eine Hülse (7088) umfasst, die den Körper (7082) verschiebbar aufnimmt.

3. Verschlusssystem (7000) gemäß Anspruch 2, wobei die Spannvorrichtung (7018) eine federnd flexible Manschette (7090) umfasst, die in der Hülse (7088) aufgenommen wird und einen Abschnitt des Körpers (7082) aufnimmt.

4. Verschlusssystem (7000) gemäß Anspruch 3, wobei die Manschette (7090) ein erstes axiales Ende, das eine ringförmige Nut in dem Körper in Eingriff nimmt, und ein zweites axiales Ende, das die Verriegelungsklemme lösbar in Eingriff nimmt, umfasst.

5. Verschlusssystem (7000) gemäß Anspruch 4, wobei die Hülse (7088) relativ zu dem Körper (7082) und der Manschette (7090) zwischen einer ersten Position, die das radiale Biegen der Manschette (7090) beschränkt, und einer zweiten Position, die gestattet, dass die Manschette relativ zu dem Körper (7082) radial nach außen gebogen wird, axial bewegbar ist.

6. Verschlusssystem (7000) gemäß Anspruch 1, wobei die Spannvorrichtung (7018) ein Einstellungsglied (7086) umfasst, das teilweise in dem Körper (7082) aufgenommen wird und den Gewindestab (7084) gewindemäßig in Eingriff nimmt, wobei das Einstellungsglied (7086) relativ zu dem Körper (7082) drehbar ist und relativ zu dem Körper (7082) axial fixiert ist, sodass eine Drehung des Einstellungsglieds (7086) relativ zu dem Körper (7082) eine entsprechende axiale Bewegung des Gewindestabs (7084) relativ zu dem Körper (7082) verursacht.

7. Verschlusssystem (7000) gemäß Anspruch 6, wobei die Spannvorrichtung (7018) einen Antidrehungsstift (7102) umfasst, der sich durch den Körper (7082) hindurch erstreckt und den Gewindestab (7084) in Eingriff nimmt, um eine relative Drehung zwischen dem Gewindestab (7084) und dem Körper (7082) zu verhindern, während eine relative axiale Bewegung zwischen dem Gewindestab (7084) und dem Körper (7082) gestattet wird.

8. Verschlusssystem (7000) gemäß Anspruch 1, wobei die Spannvorrichtung (7018) eine Kniehebel-Verriegelung (7112) umfasst, die auf dem Gewindestab (7084) montiert ist und relativ zu dem Gewindestab (7084) zwischen einer unverriegelten Position, die gestattet, dass sich das Band (7014) durch eine Apertur in dem Gewindestab (7084) hindurch bewegt, und einer verriegelten Position, in der die Kniehebel-Verriegelung das Band (7014) in Eingriff nimmt und eine Bewegung des Bands (7104) relativ zu dem Gewindestab (7084) beschränkt, drehbar ist.

9. Verschlusssystem (7000) gemäß Anspruch 1, wobei die Verriegelungsklemme (7017) ein Gehäuse (7048) und ein Nockenglied (7052) umfasst, wobei das Gehäuse den ersten Abschnitt des Bands (7024) in Eingriff nimmt, das Nockenglied (7052) relativ zu dem Gehäuse und dem ersten Abschnitt des Bands (7024) zwischen einer unverriegelten Position, die gestattet, dass sich der zweite Abschnitt des Bands (7022) durch das Gehäuse (7084) hindurch bewegt, und einer verriegelten Position, in der das Nockenglied (7052) den zweiten Abschnitt des Bands in Eingriff nimmt, um eine Bewegung des zweiten Abschnitts relativ zu dem Gehäuse (7084) und dem ersten Abschnitt (7024) zu beschränken, drehbar ist.

10. Verschlusssystem (7000) gemäß Anspruch 9, wobei das Gehäuse (7048) ein erstes Gehäuseglied (7048) und ein zweites Gehäuseglied (7050) umfasst, wobei der erste Abschnitt des Bands (7024) und das Nockenglied (7052) zwischen dem ersten und zweiten Gehäuseglied (7048, 7050) angeordnet sind.

11. Verschlusssystem (7000) gemäß Anspruch 10, wobei das erste und zweite Gehäuseglied (7048, 7050) und der erste Abschnitt des Bands (7024) allesamt Aperturen (7058, 7034, 7070) umfassen, die aufeinander ausgerichtet sind, um dem zweiten Abschnitt des Bands (7022) zu gestatten, dort hindurch zu verlaufen.

12. Verschlusssystem (7000) gemäß Anspruch 9, wobei das Gehäuse ein einstückiger, einteiliger Körper ist.

13. Verschlusssystem (7000) gemäß Anspruch 9, wobei das Nockenglied (7052) den zweiten Abschnitt (7022) des Bands (7014) gegen das Gehäuse in der verriegelten Position festklemmt.

14. Verschlusssystem (7000) gemäß Anspruch 9, wobei der erste Abschnitt des Bands (7024) eine ablenkbare Lasche (7026) umfasst, die eine Bewegung des Nockenglieds (7052) von der unverriegelten Position zu der verriegelten Position gestattet und eine Bewegung des Nockenglieds (7052) von der verriegelten Position zu der unverriegelten Position beschränkt.

15. Verschlusssystem (7000) gemäß Anspruch 9, wobei das Nockenglied (7052) eine ablenkbare Lasche umfasst, die das Gehäuse in der verriegelten Position lösbar in Eingriff nimmt, um ein Bewegen des Nockenglieds (7052) aus der verriegelten Position heraus zu beschränken.

## Revendications

1. Système de fermeture (7000) pour fixer une première partie osseuse (12a) à une seconde partie osseuse (12b), le système de fermeture comprenant :
une bande (7014) conçue pour être bouclée autour des première et seconde parties osseuses (12a, 12b) ;
une terminaison à verrouillage (7017) se mettant en prise fixe avec une première partie (7024) de la bande et se mettant sélectivement en prise fixe avec une seconde partie (7022) de la bande ; et
un dispositif de tension (7018) comprenant un corps (7082) et une tige filetée (7084), la tige filetée (7084) étant reçue dans le corps (7082) et se mettant en prise avec la bande (7014), **caractérisé en ce que** le mouvement de la tige filetée par rapport au corps (7082) dans une direction le long de l'axe longitudinal de la tige déplace la seconde partie (7022) de la bande par rapport à la première partie (7024) de la bande dans la direction le long de l'axe longitudinal de la tige.

2. Système de fermeture (7000) selon la revendication 1, ledit dispositif de tension (7018) comprenant un manchon (7088) qui reçoit de manière coulissante le corps (7082).

3. Système de fermeture (7000) selon la revendication 2, ledit dispositif de tension (7018) comprenant un collier élastiquement souple (7090) reçu dans le manchon (7088) et recevant une partie du corps (7082).

4. Système de fermeture (7000) selon la revendication 3, ledit collier (7090) comprenant une première extrémité axiale se mettant en prise avec une rainure annulaire dans le corps et une seconde extrémité axiale qui se met en prise amovible avec la terminaison à verrouillage.

5. Système de fermeture (7000) selon la revendication 4, ledit manchon (7088) étant mobile axialement par rapport au corps (7082) et au collier (7090) entre une première position limitant la flexion radiale du collier (7090) et une seconde position permettant au collier de fléchir radialement vers l'extérieur par rapport au corps (7082).

6. Système de fermeture (7000) selon la revendication 1, ledit dispositif de tension (7018) comprenant un élément de réglage (7086) partiellement reçu dans le corps (7082) et se mettant en prise par vissage avec la tige filetée (7084), l'élément de réglage (7086) pouvant pivoter par rapport au corps (7082) et étant axialement fixe par rapport au corps (7082) de sorte que la rotation de l'élément de réglage (7086) par rapport au corps (7082) provoque un mouvement axial correspondant de la tige filetée (7084) par rapport au corps (7082).

7. Système de fermeture (7000) selon la revendication 6, ledit dispositif de tension (7018) comprenant une goupille anti-rotation (7102) s'étendant à travers le corps (7082) et se mettant en prise avec la tige filetée (7084) pour limiter la rotation relative entre la tige filetée (7084) et le corps (7082) tout en permettant un mouvement axial relatif entre la tige filetée (7084) et le corps (7082).

8. Système de fermeture (7000) selon la revendication 1, ledit dispositif de tension (7018) comprenant une fermeture à genouillère (7112) montée sur la tige filetée (7084) et mobile par rapport à la tige filetée (7084) entre une position déverrouillée permettant à la bande (7014) de se déplacer à travers un orifice dans la tige filetée (7084) et une position verrouillée dans laquelle la fermeture à genouillère se met en prise avec la bande (7014) et limite le mouvement de la bande (7104) par rapport à la tige filetée (7084).

9. Système de fermeture (7000) selon la revendication 1, ladite terminaison à verrouillage (7017) comprenant un logement (7048) et un élément de came (7052), ledit logement se mettant en prise avec la première partie de la bande (7024), ledit élément de came (7052) pouvant pivoter par rapport au logement et à la première partie de la bande (7024) entre une position déverrouillée permettant à la seconde partie de la bande (7022) de se déplacer à travers le logement (7084) et une position verrouillée dans laquelle l'élément de came (7052) se met en prise avec la seconde partie de la bande pour limiter le mouvement de la seconde partie par rapport au logement (7084) et à la première partie (7024).

10. Système de fermeture (7000) selon la revendication 9, ledit logement (7048) comprenant un premier élément de logement (7048) et un second élément de logement (7050), la première partie de la bande (7024) et ledit élément de came (7052) étant disposés entre le premier et le second élément de logement (7048, 7050).

11. Système de fermeture (7000) selon la revendication 10, lesdits premier et second éléments de logement (7048, 7050) et ladite première partie de la bande (7024) comportant tous des orifices (7058, 7034, 7070) qui sont alignés les uns par rapport aux autres pour permettre à la seconde partie de la bande (7022) de passer à travers.

12. Système de fermeture (7000) selon la revendication 9, ledit logement étant un corps unitaire monobloc.

13. Système de fermeture (7000) selon la revendication 9, ledit élément de came (7052) serrant la seconde partie (7022) de la bande (7014) contre le logement dans la position verrouillée.

14. Système de fermeture (7000) selon la revendication 9, ladite première partie de la bande (7024) comprenant une patte déformable (7026) qui permet le mouvement de l'élément de came (7052) de la position déverrouillée à la position verrouillée et limite le mouvement de l'élément de came (7052) de la position verrouillée à la position déverrouillée.

15. Système de fermeture (7000) selon la revendication 9, ledit élément de came (7052) comprenant une patte déformable qui se met en prise amovible avec le logement dans la position verrouillée pour empêcher l'élément de came (7052) de se déplacer hors de la position verrouillée.
